# EUROPÄISCHE PATENTSCHRIFT

(11) **EP 1 727 523 B1**
(45) Veröffentlichungstag und Bekanntmachung des Hinweises auf die Patenterteilung: **23.11.2016**
(21) Anmeldenummer: 05737707.9
(22) Anmeldetag: 17.03.2005
(51) Int. Cl.: A61K 9/00, A61K 9/48

(54) **PHARMAZEUTISCHE FORMULIERUNG MIT UNVERMAHLENEM FLUTAMID**
PHARMACEUTICAL FORMULATION CONTAINING UNMILLED FLUTAMIDE
FORMULATION PHARMACEUTIQUE COMPRENANT UN FLUTAMIDE NON MOULU

(30) Priorität: 22.03.2004 DE 102004014272
(43) Veröffentlichungstag der Anmeldung: 06.12.2006
(73) Patentinhaber: Hexal AG, 83607 Holzkirchen (DE)
(72) Erfinder: RUNGE, Hans-Joachim, 39291 Hohenwarte (DE); LEMBCKE, Adalbert, 39326 Colbitz OT Lindhorst (DE)
(74) Vertreter: Forstmeyer, Dietmar
(86) Internationale Anmeldenummer: PCT/EP2005/002884
(87) Internationale Veröffentlichungsnummer: WO 2005/089711

(56) Entgegenhaltungen:
- EP-A- 0 348 564
- EP-A- 0 543 541
- WO-A-98/47499
- US-A- 3 995 060
- US-A- 4 474 813
- US-B1- 6 228 401
- AOKI M ET AL: "Flutamide composition containing nonionic surfactant and opt. fatty acid ester of lower poly-hydric alcohol" DATABASE WPI, 1997, XP002978066

## Beschreibung

Die Erfindung bezieht sich auf eine pharmazeutische Formulierung mit kristallinem und/oder amorphem unvermahlenen Flutamid sowie ein Verfahren zu deren Herstellung.

Flutamid bezeichnet die chemische Verbindung 4'-Nitro-3'-trifluormethylisobutyranilid oder 2-Methyl-N-[4-nitro-3-(trifluoromethyl)phenyl]propanamid (CAS 13311-84-7). Die chemische Synthese von Flutamid wird bei Baker et al., J. Med. Chem. 10, S. 93 (1967), in US 3,847,988, US 3,995,060, DE 21 30 450 sowie in DE 22 61 293 beschrieben. Flutamid ist eine nicht steroidale Verbindung, die sich insbesondere durch antiandrogene Eigenschaften auszeichnet (vgl. Martindale, "The Extra Pharmacopoeia", 30. Aufl. (1993), S. 482; DE 21 30 450, DE 22 61 293; US 3,847,988). Flutamid wird zur palliativen Behandlung des Prostatacarcinoms eingesetzt. Bei oraler Applikation beträgt die empfohlene Tagesdosis 750 mg. Allgemein wird diese Dosis in Form von 3 Teilmengen von jeweils 250 mg über den Tag verteilt. Bei Tabletten ist die empfohlene Wirkstoffmenge gewöhnlich in einer Tablette enthalten, bei Kapseln ist diese auf zwei mit jeweils 125 mg Wirkstoff verteilt. Flutamid stellt ein Prodrug dar, sein pharmakologisch aktiver Metabolit ist "2-Hydroxyflutamid".
Flutamid ist nahezu unlöslich in Wasser sowie in saurem Milieu, beispielsweise in 0.1%-iger Salzsäure, deren pH-Wert dem des Magensaftes entspricht. Aufgrund seiner Wasserunlöslichkeit und der notwendigen hohen oralen Dosis ist die Bioverfügbarkeit von Flutamid stark von der Partikelgröße des Wirkstoffs abhängig. Dieser Sachverhalt läßt sich wie folgt erklären: zunächst muß der Wirkstoff aus der Arzneiform freigesetzt werden und vorteilhafterweise im Verdauungssaft gelöst oder fein verteilt vorliegen, bevor er durch die Membran der Schleimhäute permeieren und vom Organismus aufgenommen werden kann.
In der Literatur wird die Herstellung von pharmazeutischen Zubereitungen mit Flutamid unter Verwendung besonders kleiner Wirkstoff-Teilchen beschrieben.

DE 21 30 450 beschreibt den Einsatz von Flutamid als Tierarzneimittel, wobei der Wirkstoff feinvermahlen eingesetzt wird.

US 3,995,060 offenbart die Herstellung von Tabletten, parenteralen Suspensionen sowie Kapseln mit Flutamid. Der Wirkstoff wird chemisch synthetisiert, isoliert und beispielsweise durch Umkristallisieren gereinigt (Spalte 2 Zeile 37). Als Ausgangsmaterial für die Herstellung von Kapseln wird das Flutamid mit Trockeneisbrocken gemischt und bis zu einer Teilchengröße von 240 bis 5 µm gemahlen (Tabelle Spalte 17), danach mit unter anderem Natriumlaurylsulfat (SLS) in einem Gewichtsverhältnis Flutamid : SLS = 1,0 : 1,2 bis 1,0 : 0,06 (Tabelle Spalte 16) gemischt und danach einem weiteren Mahlvorgang unterworfen (Spalte 17 Zeilen 17 bis 20).

Obgleich gemäß US 3 995 060 das durch Umkristallisieren gereinigte Flutamid, noch bevor es mit irgendeinem weiteren Stoff zusammengebracht wird, einem ersten Mahlvorgang unterworfen und nach dem Vermischen mit unter anderem Natriumlaurylsulfat einem weiteren Mahlvorgang unterworfen wird, wird in US 6,187,345 (Spalte 1 Zeilen 30 bis 43) sowie US 6,228,401 (Spalte 1 Zeilen 38 bis 51) gerügt, daß mit Blick auf eine adäquate Bioverfügbarkeit US 3 995 060 die Wirkstoffzerkleinerung nicht ausreichend kritisch gesehen habe. US 6 187 345 und US 6 228 401 beschreiben die Herstellung von Flutamidpartikeln mit einer spezifischen Oberfläche von 0.40 - 2.50 m²/cm³ (Spalte 2 Zeile 67 bzw. Spalte 3 Zeile 7) und einer Partikelgröße von 10 bis 130 *µ*m (Spalte 3 Zeile 4 bzw. Splate 3 Zeilen 11 bis 12). Hierfür wird Flutamid in Gegenwart von beispielsweise Lactose als Streckmitteln vermahlen (Beispiele 1 bis 4). Das Streckmittel verhindert eine Agglomeration des Flutamids beim Mahlprozess. Das so erhaltene mikronisierte Flutamid mit einer bestimmten Partikelgröße und spezifischen Oberfläche soll eine adäquate Bioverfügbakeit zeigen.
Zur Herstellung von Kapseln wird das erhaltene Gemisch aus Flutamid und Lactose mit unter anderem Natriumlaurylsulfat (SLS) feucht granuliert (Beispiele 6, 7 und 9), wobei das Gewichtsverhältnis Flutamid : SLS = 1,0 : 0,096 bis 1,0 : 0,06 beträgt.

In "Dissolution rate limited bioavailability of flutamide, and in vitro - in vivo correlation", J. Posti et al., Eur. J. Pharm. Biopharm., 49 (2000), 35 - 39, wird gezeigt, daß die Verwendung von unvermahlenem Flutamid in Tabletten nach 45 min. eine Wirkstofffreisetzung von 45 % ergibt, während beim Einsatz von fein vermahlenem Flutamid eine Freisetzungsrate von 90 % in der gleichen Zeit erreicht wird. Weiterhin wird bewiesen, daß die Freisetzung des Wirkstoffes in vitro quantitativ mit seiner Bioverfügbarkeit korreliert. Als Maß wird hierbei der AUC-Wert des eigentlich wirksamen Metaboliten 2-Hydroxyflutamid herangezogen. Diese Verfahrensweise ist allgemein anerkannt, so daß bei der Bestimmung der in vitro Werte zuverlässig auf die Bioverfügbarkeit des Flutamids in seinen Zubereitungen geschlossen werden kann. Eine Untersuchung von 25 Chargen von 250 mg Flutamid-Tabletten verschiedener Hersteller aus dem Zeitraum von 1987 bis 1994 ergab, daß diese Produkte in ihrer jeweiligen Freisetzungsrate (nach 45 min.) im Bereich von 47 - 94 % variieren. Zudem schwanken die Freisetzungen von verschiedenen Chargen beim gleichen Hersteller innerhalb von 47 - 86 %. Aufgrund der großen Wertestreuung der Freisetzungsraten können die bisherigen Herstellungsprozesse nicht als valid bezeichnet werden. Eine ausreichende klinische Sicherheit ist nicht gegeben. Ursache sind die schwer beherrschbaren Stoffeigenschaften von Flutamid.

Posti et al beschreiben in Europ. J. Pharm. Biopharm., 49 (2000) 35-39 übliche Tabletten mit gemahlenem oder ungemahlenem Flutamid zur unmittelbaren Wirkstoff-Freisetzung und mit einem Gehalt an Maisstärke, mikrokristalliner Zellulose, kolloidalem wasserfreien Siliziumdioxid und Magnesiumstearat.

Schließlich werden in US 6,228,401 (Test Procedure I) Kapseln beschrieben, die unvermahlenes Flutamid enthalten. Die damit erzielte Wirkstoff-Konzentration im Plasma wird mit einer Konzentration verglichen, die mit Eulexin^{®} als Handelsprodukt erreichbar ist.

Nachteilig an einem Vermahlungsprozess für Flutamid ist die für den Mahlvorgang erforderliche, kostenintensive Kühlung. Die Prozesswärme, welche beim Vermahlen von Flutamid entsteht, muß abgeführt werden, da es auf Grund des niedrigen Schmelzpunktes von Flutamid (111 °C) sonst zu Schmelz- oder Sintererscheinungen kommen kann. Eine Herstellung von micronisiertem Flutamid, beipielsweise in Luftstrahlmühlen, ist mit großem Kühlaufwand zwar möglich, jedoch neigt das so vermahlene Flutamid zur Bildung von Agglomeraten. Deshalb ist auch ein längeres Aufbewahren von micronisiertem Flutamid nur bedingt möglich.

Aufgabe der Erfindung ist die Bereitstellung von pharmazeutischen Formulierungen mit kristallinem und/oder amorphem unvermahlenem Flutamid. Die Formulierungen sollen eine reproduzierbare und hohe Freisetzungsrate für den Wirkstoff aufweisen. Die Herstellung dieser Formulierungen soll kostengünstig sein.

Gemäß einer Ausführungsform wird die der Erfindung zugrundeliegende Aufgabe durch eine pharmazeutische Formulierung mit kristallinem und/oder amorphem unvermahlenen Flutamid im Gemisch mit mindestens einer oberflächenaktiven Substanz gelöst.

So betrifft die Erfindung eine pharmazeutische Formulierung mit kristallinem und/oder amorphem unvermahlenen Flutamid im Gemisch mit mindestens einer oberflächenaktiven Substanz, wobei die Formulierung in Form einer Tablette mit einem Gehalt an mindestens einem Fließregulierungsmittel vorliegt.

So betrifft die Erfindung ferner eine pharmazeutische Formulierung mit kristallinem und/oder amorphem unvermahlenen Flutamid im Gemisch mit mindestens einer oberflächenaktiven Substanz, wobei die Formulierung als Kapsel-Abfüllung vorliegt.

So betrifft die Erfindung ferner eine pharmazeutische Formulierung mit kristallinem und/oder amorphem unvermahlenen Flutamid im Gemisch mit mindestens einer oberflächenaktiven Substanz, wobei die Formulierung als Dragee, Brausetablette, Zäpfchen oder Granulat vorliegt.

Die erfindungsgemäße Formulierung kann mit Flutamid einer Teilchengröße vorgesehen sein, wie sie bei der Synthese mit nachgeschaltetem(n) Reinigungsschritt(en) anfällt.

Ferner kann die erfindungsgemäße Formulierung mit Flutamid einer Teilchengröße vorgesehen sein, wie sie bei der Synthese mit einer Umkristallisation als nachgeschaltetem Reinigungsschritt anfällt.

Ferner kann die erfindungsgemäße Formulierung mit Flutamid einer mittleren Teilchengröße vorgesehen sein, die größer als die mittlere Teilchengröße von Flutamid ist, das bei einer Ausgangsteilchengröße von 5 bis 240 *µ*m einem Mahlvorgang unterworfen worden ist.

Ferner kann die erfindungsgemäße Formulierung mit Flutamid einer Größe von 50 % der Teilchen (X50-Wert) größer 20 *µ*m vorgesehen sein.

Ferner kann die erfindungsgemäße Formulierung mit Flutamid einer Größe von 50 % der Teilchen (X50-Wert) größer 26 *µ*m vorgesehen sein.

Ferner kann die erfindungsgemäße Formulierung mit Flutamid einer Größe von 90 % der Teilchen (X90-Wert) größer 60 *µ*m vorgesehen sein.

Ferner kann die erfindungsgemäße Formulierung mit Flutamid einer Größe von 90 % der Teilchen (X90-Wert) größer 130 *µ*m vorgesehen sein.

Ferner kann die erfindungsgemäße Formulierung mit Flutamid einer spezifischen Oberfläche kleiner 2,50 m²/cm³ vorgesehen sein.

Ferner kann die erfindungsgemäße Formulierung mit Flutamid einer spezifischen Oberfläche kleiner 1,50 m²/cm³ vorgesehen sein.

Ferner kann die erfindungsgemäße Formulierung mit Flutamid einer spezifischen Oberfläche kleiner 0,35 m²/cm³ vorgesehen ein.

Ferner kann die erfindungsgemäße Formulierung mit Flutamid und/oder einem seiner pharmazeutisch unbedenklichen Solvate vorgesehen sein.

Ferner kann die erfindungsgemäße Formulierung mit mindestens einer oberflächenaktiven Substanz vorgesehen sein, die aus der durch
- anionenaktive Verbindungen,
- kationenaktive Verbindungen und
- nichtionogene Tenside
gebildeten Gruppe ausgewählt ist.

Ferner kann die erfindungsgemäße Formulierung mit Natriumdodecylsulfat als oberflächenaktiver Substanz vorgesehen sein.

Ferner kann die erfindungsgemäße Formulierung mit einem Gewichtsverhältnis Flutamid : oberflächenaktive Substanz(en) von 5 : 1 bis 30 : 1 vorgesehen sein.

Ferner kann die erfindungsgemäße Formulierung mit einem Gewichtsverhältnis Flutamid : oberflächenaktive Substanz(en) von 5 : 1 bis 20 : 1 vorgesehen sein.

Ferner kann die erfindungsgemäße Formulierung mit einem Gewichtsverhältnis Flutamid : oberflächenaktive Substanz(en) von 10 : 1 bis 15 : 1 vorgesehen sein.

Ferner kann die erfindungsgemäße Formulierung als formlose Mischung oder als Gegenstand vorgesehen sein, der einer Formgebung unterworfen worden ist, beispielsweise in Form einer Tablette, einer Kapsel-Abfüllung, eines Dragees, einer Brausetablette, eines Zäpfchens oder eines Granulats.

Ferner kann die erfindungsgemäße Formulierung mit einem Gehalt an 50 bis 2000 mg Flutamid vorgesehen sein.

Ferner kann die erfindungsgemäße Formulierung mit einem Gehalt an 50 bis 500 mg Flutamid vorgesehen sein.

Ferner kann die erfindungsgemäße Formulierung mit einem Gehalt an 100 bis 200 mg Flutamid vorgesehen sein.

Ferner kann die erfindungsgemäße Formulierung mit einem Gehalt an mindestens einem Hilfsstoff aus der durch anorganische Füllstoffe, organische Füllstoffe, Bindemittel, Gleitmittel, Schmiermittel, Fließreguliermittel und/oder Sprengmittel gebildeten Gruppe vorgesehen sein.

Ferner kann die erfindungsgemäße Formulierung mit einem Gehalt an mindestens einem weiteren pharmazeutischen Wirkstoff neben Flutamid vorgesehen sein.

Gemäß einer weiteren Ausführungsform wird die der Erfindung zugrundeliegende Aufgabe durch ein Verfahren zur Herstellung einer pharmazeutischen Formulierung mit kristallinem und/oder amorphem unvermahlenen Flutamid im Gemisch mit mindestens einer oberflächenaktiven Substanz gelöst, bei dem man das Flutamid mit der mindestens einen oberflächenaktiven Substanz einem intensiven Mischprozeß unterwirft, vorzugsweise in einem Zwangsmischer mischt, und die anfallende Mischung zu einer erfindungsgemäßen Formulierung weiterverarbeitet.

Bei dem erfindungsgemäßen Verfahren kann man bei einer Temperatur von 0 bis 40 °C mischen.

Ferner kann man bei dem erfindungsgemäßen Verfahren bei Raumtemperatur mischen.

Ferner kann man bei dem erfindungsgemäßen Verfahren einen oder mehrere Hilfsstoffe mit einem Zwangsmischer oder mit einem Freifallmischer zumischen.

Ferner kann man bei dem erfindungsgemäßen Verfahren bei dem man einen oder mehrere weitere pharmazeutische Wirkstoffe, bei denen es sich nicht um Flutamid handelt, mit einem Zwangsmischer oder mit einem Freifallmischer zumischen.

Ferner kann man bei dem erfindungsgemäßen Verfahren die erhaltene Mischung zu Tabletten, Kapseln, Dragees, Brausetabletten, Zäpfchen oder Granulat weiterverarbeiten.

Ferner kann man bei dem erfindungsgemäßen Verfahren die erhaltene Mischung einer Direkttablettierung unterwerfen.

Schließlich kann man bei dem erfindungsgemäßen Verfahren Granulat zu Tabletten weiterverarbeiten.

Die der Erfindung zugrundeliegende Aufgabe wird außerdem durch eine pharmazeutische Formulierung mit kristallinem und/oder amorphem unvermahlenen Flutamid im Gemisch mit mindestens einer oberflächenaktiven Substanz gelöst, die nach einem erfindungsgemäßen Verfahren herstellbar ist.

Überraschenderweise wurde also nun gefunden, daß sich unvermahlenes Flutamid zu pharmazeutischen Formulierungen mit hohen und reproduzierbaren Freisetzungsraten verarbeiten läßt, wenn die Formulierung auf einer Pulvermischung mit kristallinem und/oder amorphem Flutamid und mindestens einer oberflächenaktiven Substanz basiert. Zur Herstellung der erfindungsgemäßen Pulvermischung werden unvermahlenes Flutamid, mindestens eine oberflächenaktive Substanz und ggf. weitere Hilfsstoffe in einem Zwangsmischer intensiv gemischt. Diese Pulvermischung kann beispielsweise zu Tabletten, Kapseln, Dragees, Brausetabletten oder Zäpfchen weiterverarbeitet werden. Die so erhaltenen pharmazeutischen Formulierungen zeigen nicht nur eine reproduzierbare, sondern auch eine höhere Freisetzungsrate für Flutamid als Formulierungen mit micronisiertem Wirkstoff. Die Herstellung mit Hilfe eines Zwangsmischers ist kostengünstiger und zeitsparender verglichen mit einem zusätzlichen aufwändigen Vermahlungsprozess unter Materialeinbußen.

Für die erfindungsgemäße pharmazeutische Formulierung kann Flutamid in Form des freien Säureamids und/oder eines seiner Solvate eingesetzt werden.

Die Tagesdosis von Flutamid beträgt 2 - 30 mg pro kg Körpergewicht des Patienten, bevorzugt 7 - 14 mg/kg Körpergewicht. Eine Tagesdosis kann auf mehrere Gaben pro Tag aufgeteilt werden. Eine pharmazeutische Formulierung kann 100 bis 2000 mg Flutamid enthalten.

Mit dem Begriff "unvermahlenes Flutamid" ist kristallines oder amorphes Flutamid gemeint, welches gewöhnlich in einer der Synthese nachgeschalteten Reinigungsoperation (z. B. Umkristallisation) anfällt.

Als oberflächenaktive Substanzen eignen sich
- anionenaktive Verbindungen wie Natriumdodecylsulfat oder Natriumdocusat (Stearate werden im Rahmen der Erfindung als Gleitmittel, Schmiermittel oder Fließregulierungsmittel verstanden; vgl. auch Hunnius, Pharmazeutisches Wörterbuch, 8. Auflage, De Gruyter, für die Begriffe Gleitmittel, Schmiermittel und Fließmittel bzw. Fließregulierungsmittel),
- kationenaktive Verbindungen wie Triethanolaminoleat,
- nichtionogene Tenside, beispielsweise Cetylalkohol, Polysorbat, Glycerylmonostearat, Glycerylmonooleat, Polyvinylalkohol, Tweens® wie Sorbitan-monoisostearat, Sorbitan-monolaurat, Sorbitan-monopalmitat, Sorbitanmonostearat, Sorbitan-monooleat, Sorbitan-sequioleat oder Sorbitan-trioleat.
Es können auch Mischungen mit mehreren Tensiden eingesetzt werden.
Bevorzugt wird Natriumdodecylsulfat eingesetzt.

Das Gewichtsverhältnis von Flutamid zu der (den) oberflächenaktiven Substanz(en) beträgt
5 : 1 bis 30 : 1, insbesondere 10 : 1 bis 15 : 1.

Für die Herstellung der Pulvermischung können folgende Hilfsstoffe eingesetzt werden:
- Füllstoffe wie Cellulose (z. B. mikrokristalline Cellulose), Cellulosederivate, Zucker (z. B. Lactose, Glucose, Saccharose), Zuckeralkohole (z. B. Mannitol, Sorbitol), Stärke (z. B. Kartoffel-, Weizen-, Mais- und/oder Reisstärke),
- Gleitmittel wie Siliciumdioxid, Calcium-, Aluminium- und/oder Magensiumstearat,
- Schmiermittel wie Aluminium-, Magnesium- oder Calciumstearat, Stearinsäure, Natriumstearylfumarat, Polyethylenglycol, Stearyl- oder Cetylalkohol, Palmitinsäure, hydrierte Pflanzenöle und/oder Talkum,
- Sprengmittel wie Stärke (z. B. Kartoffel- oder Maisstärke), Polyvinylpyrrolidone (Kollidone, quervernetztes Polyvinylpyrrolidon), unmodifizierte oder modifizierte Cellulose (z. B. mikrokristalline Cellulose, Natriumcarboxymethylcellulose, quervernetzte Carboxymethylcellulose), Ultraamylopektin, Formaldehydgelatine und/oder Alginsäure oder ihre Alginate Wird die erfindungsgemäße Pulvermischung zur Befüllung von Kapseln weiter verwendet, so werden bevorzugt folgende Hilfsstoffe für die Herstellung der Pulvermischung eingesetzt:
- Lactose, mikrokristalline Cellulose und Maisstärke als Füllstoffe,
- Magnesiumstearat und Siliciumdioxid als Gleitmittel

Wird die erfindungsgemäße Pulvermischung zu Tabletten weiterverarbeitet, so werden bevorzugt folgende Hilfsstoffe für die Herstellung der Pulvermischung eingesetzt:
- Lactose und mikrokristalline Cellulose als Füllstoffe,
- Maisstärke und mikrokristalline Cellulose als Sprengmittel,
- Siliciumdioxid als Gleitmittel
- Magnesiumstearat als Schmiermittel bzw. Fließregulierungsmittel

Für die Herstellung der erfindungsgemäßen Pulvermischung wird unvermahlenes Flutamid mit mindestens einer oberflächenaktiven Substanz in einem Zwangsmischer einem intensiven Mischprozess unterworfen. Weitere Hilfsstoffe können in einem zusätzlichen Freifallmischer oder im gleichen Zwangsmischer nacheinander oder gleichzeitig zugemischt werden.

Unvermahlenes Flutamid kann auch zusammen mit mindestens einer oberflächenaktiven Substanz sowie einem oder mehreren Hilfsstoffen in einem Zwangsmischer intensiv gemischt werden.

Für das erfindungsmäße Verfahren können konventionelle Zwangsmischer mit Edelstahl-Innenseite verwendet werden. Zwangsmischer sind im allgemeinen Mischer mit rundem oder flachem Boden und nahe dem Boden rotierenden Flügeln oder Schaufeln. Sie können sog. "chopper", d.h. schnell rotierende Messer haben, die in das Mischgefäß hineinragen. Die Rotationsgeschwindigkeit der Flügel kann 100 - 800 rpm betragen, die der "chopper" 750 - 3000 rpm. Bevorzugt wird eine Rotationsgeschwindigkeit der Flügel von 150 - 200 rpm bzw. der "chopper" von 1500 rpm eingesetzt. Als Zwangsmischer kann beispielsweise ein Collette Vactron®-, Lödige®- oder Diosna®-Mischer oder ein Bohle-Vagumat® verwendet werden.

Der Zwangsmischprozess kann bei unterschiedlichen Temperaturen durchgeführt werden. Bevorzugt ist aus wirtschaftlichen Gründen jedoch Raumtemperatur. Die Prozesszeit kann 1 - 180 min., insbesondere 3 bis 60 min. betragen.

Die erfindungsgemäße Pulvermischung mit unvermahlenem Flutamid kann zur Herstellung pharmazeutischer Darreichungsformen, beispielsweise Kapseln, Tabletten, Brausetabletten, Dragees, Suspensionen oder Zäpfchen verwendet werden. Diese Darreichungsformen können auch Hilfsstoffe wie Trägermaterialien, Füllstoffe, Bindemittel, Feuchthaltemittel, Gleitmittel, Konservierungsmittel, Stabilisatoren, Aromen und/oder Farbpigmente enthalten.

### Weiterverarbeitung zu Kapseln:

Die erfindungsgemäße Pulvermischung mit unvermahlenem Flutamid kann in Kapseln gefüllt werden.

### Weiterverarbeitung zu Tabletten:

Die erfindungsgemäße Pulvermischung mit unvermahlenem Flutamid kann einer Direkttablettierung unterworfen werden. Sie kann aber auch zu Granulaten verarbeitet werden, die, mit weiteren Hilfsstoffen gemischt, zu Tabletten verpresst werden können.

Als Hilfsstoffe bei der Granulat- bzw. Tablettenherstellung eignen sich z. B.:
- organische Füllstoffe wie Cellulose und/oder Cellulosederivate (z. B. mikrokristalline Cellulose), Zucker (z. B. Lactose, Glucose, Saccharose), Zuckeralkohole (z. B. Mannitol, Sorbitol), Stärke (z. B. Kartoffel-, Weizen-, Mais- und/oder Reisstärke),
- anorganische Füllstoffe wie Calciumphosphat oder -sulfat, Natriumcalciumphosphat, Natriumchlorid oder Kaolin,
- Bindemittel wie Gelatine-, Stärke- oder Cellulosederivate (z. B. Hydroxypropylcellulose, Hydroxypropylmethylcellulose, Methylcellulose, Natriumcarboxymethylcellulose), Polyvinylpyrrolidone, Zucker (z. B. Sucrose, Glucose, Dextrose), natürliche Gummi (z. B. Natriumalginat, arabisches Gummi, Tragant, Pektin),
- Schmiermittel wie Magnesiumstearat, Calciumstearat, Stearinsäure, Natriumstearylfumarat, Polyethylenglycol, Palmitinsäure, hydrierte Pflanzenöle und/oder Talkum,
- Fließregulierungsmittel wie Talkum, Stearinsäure und ihre Erdalkalisalze, Siliziumdioxid, Polyethylenglykole und langkettige Alkohole,
- Sprengmittel wie Stärke und -derivate (Maisstärke, Natriumcarboxymethylstärke), quervernetztes Polyvinylpyrrolidon, unmodifizierte oder modifizierte Cellulose (z. B. Natriumcarboxy-methylcellulose, quervernetzte Carboxymethylcellulose), Alginsäure und ihre Alginate, Calciumcarbonat und/oder Natriumbicarbonat, oberflächenaktive Stoffe (z. B. Syndets).

Die Tabletten können mit einem filmbildenden Material überzogen werden, um z. B. Magensaftresistenz zu erreichen, eine glatte Oberfläche zu erhalten oder um die Stabilität der Tablette während der Verpackung und dem Transport zu erhöhen. Dieser Tablettenüberzug kann beispielsweise Additive wie "antitacking" Materialien oder Farbstoffe enthalten.

### Freisetzungsprofil:

In vitro Freisetzungsstudien für Kapseln oder Tabletten mit Flutamid wurden mit einer Vankel-Apparatur mit Blattrührer durchgeführt. Als Freisetzungsmedium wurde eine 2%-ige Natriumlaurylsulfat-Lösung bei einem pH-Wert von 5,5 - 7,5 verwendet.

Die Erfindung wird durch nachstehende Beispiele näher erläutert, ohne aber den Erfindungsumfang damit einzuschränken.

### Beispiel 1:

Die folgenden Stoffe werden zur Herstellung von Flutamid-Kapseln verwendet.

| **Bestandteile** | **Prozent (%)** | **Gewicht (mg/Kapsel)** |
|---|---|---|
| Flutamid, kristallin, unvermahlen | 33,2 | 80,0 |
| Lactose | 29,4 | 70,9 |
| Natriumdodecylsulfat | 2,0 | 4,8 |
| Mikrokristalline Cellulose | 13,26 | 32,0 |
| Maisstärke | 21,6 | 52,0 |
| Siliciumdioxid | 0,04 | 0,1 |
| Magnesiumstearat | 0.5 | 1,2 |
| Gesamt | 100 | 241 |

### Herstellung:

Lactose, Natriumdodecylsulfat, mikrokristalline Cellulose und kristallines Flutamid werden in einem Zwangsmischer 3 min. lang intensiv gemischt. Danach werden Maisstärke, kolloidales Siliciumdioxid und Magnesiumstearat zugegeben und die Mischung für 5 min. in einem Freifallmischer homogenisiert. Von dieser Mischung werden je 241 mg in Hartgelatinekapseln gefüllt.

### Beispiel 2 (Vergleich):

Die folgenden Stoffe werden zur Herstellung von Flutamid-Kapseln verwendet.

| **Bestandteile** | **Prozent (%)** | **Gewicht (mg/Kapsel)** |
|---|---|---|
| Flutamid, micronisiert | 33.2 | 80.0 |
| Lactose | 29.4 | 70.9 |
| Natriumdodecylsulfat | 2.0 | 4.8 |
| Mikrokristalline Cellulose | 13.26 | 32.0 |
| Maisstärke | 21.6 | 52.0 |
| Siliciumdioxid | 0.04 | 0.1 |
| Magnesiumstearat | 0.5 | 1.2 |
| Gesamt | 100 | 241 |

### Herstellung:

Lactose, Natriumdodecylsulfat, mikrokristalline Cellulose, Maisstärke, kolloidales Siliciumdioxid und Magnesiumstearat werden für 15 min. mit micronisiertem Flutamid in einem Freifallmischer gemischt. Von dieser Mischung werden je 241 mg in Hartgelatinekapseln gefüllt.

### Beispiel 3:

Die folgenden Stoffe werden zur Herstellung von Flutamid-Kapseln verwendet.

| **Bestandteile** | **Prozent (%)** | **Gewicht (kg/100.000 Kapseln)** |
|---|---|---|
| Flutamid, kristallin, unvermahlen | 33.0 | 12.446 |
| Lactose | 29.3 | 11.047 |
| Natriumdodecylsulfat | 2.0 | 0.747 |
| Mikrokristalline Cellulose | 13.2 | 4.981 |
| Maisstärke | 21.9 | 8.272 |
| Siliciumdioxid | 0.1 | 0.020 |
| Magnesiumstearat | 0.5 | 0.188 |
| Gesamt | 100 | 37.701 |

### Herstellung:

Lactose, Natriumdodecylsulfat, mikrokristalline Cellulose und kristallines Flutamid werden in einem Zwangsmischer 40 min. lang intensiv gemischt. Danach werden Maisstärke, kolloidales Siliciumdioxid und Magnesiumstearat zugegeben und die Mischung für 3 min. bei verminderter Drehzahl homogenisiert. Von dieser Mischung werden je 377 mg in Hartgelatinekapseln gefüllt.

### Beispiel 4:

Die folgenden Stoffe werden zur Herstellung von Flutamid-Tabletten verwendet.

| **Bestandteile** | **Prozent (%)** | **Gewicht (kg)** |
|---|---|---|
| Flutamid, kristallin, unvermahlen | 33.2 | 1.658 |
| Lactose | 29.4 | 1.471 |
| Natriumdodecylsulfat | 2.0 | 0.100 |
| Mikrokristalline Cellulose | 13.2 | 0.663 |
| Maisstärke | 21.6 | 1.08 |
| Siliciumdioxid | 0.1 | 0.003 |
| Magnesiumstearat | 0.5 | 0.025 |
| Gesamt | 100 | 5.000 |

### Herstellung:

Kristallines Flutamid, Lactose, Natriumdodecylsulfat und mikrokristalline Cellulose werden im Diosna-Mischer 10 min. zwangsgemischt. Anschließend wird Maisstärke zugegeben und wieder für 5 min. zwangsgemischt. Nach der Zugabe von Siliciumdioxid und Magnesiumstearat wird für 3 min. endgemischt.

Aus dieser Mischung werden auf einer Rundläuferpresse Tabletten mit 750 mg Gewicht direktgepreßt. (Härte 98 N, Abrieb < 1 %)

### Beispiel 5 (Vergleich) :

Die folgenden Stoffe werden zur Herstellung von Flutamid-Tabletten verwendet.

| **Bestandteile** | **Prozent (%)** | **Gewicht (kg)** |
|---|---|---|
| Flutamid, kristallin, unvermahlen | 33.0 | 21,250 |
| Lactose | 29.3 | 18,850 |
| Natriumdodecylsulfat | 2.0 | 1,275 |
| Mikrokristalline Cellulose | 13.2 | 8,500 |
| Maisstärke | 21.5 | 13,820 |
| Siliciumdioxid | 0.5 | 0,340 |
| Magnesiumstearat | 0.5 | 0,340 |
| Gesamt | 100 | 64,375 |

### Herstellung:

Lactose, Natriumdodecylsulfat, mikrokristalline Cellulose, Maisstärke und kristallines Flutamid werden mit 21.080 l Wasser granuliert, getrocknet, gesiebt, anschließend mit kolloidalem Siliciumdioxid und Magnesiumstearat vermischt und zu Tabletten verpresst.

### Beispiel 6 (Vergleich):

Die folgenden Stoffe werden zur Herstellung von Flutamid-Tabletten verwendet.

| **Bestandteile** | **Prozent (%)** | **Gewicht (kg)** |
|---|---|---|
| Flutamid, micronisiert | 33.0 | 21,250 |
| Lactose | 29.3 | 18,850 |
| Natriumdodecylsulfat | 2.0 | 1,275 |
| Mikrokristalline Cellulose | 13.2 | 8,500 |
| Maisstärke | 21.5 | 13,820 |
| Siliciumdioxid | 0.5 | 0,340 |
| Magnesiumstearat | 0.5 | 0,340 |
| Gesamt | 100 | 64,375 |

Die Herstellung der Tabletten erfolgt analog Beispiel 5.

### Freisetzungsraten für die Beispiele 1-6:

Die Freisetzungsrate von Flutamid aus den pharmazeutischen Formulierungen mit
- unvermahlenem Flutamid intensiv gemischt mit Natriumdodecylsulfat (Beispiele 1, 3, 4),
- mikronisiertem Flutamid, nicht intensiv gemischt mit Natriumdodecylsulfat (Beispiel 2), Mengenverhältnisse wie in Beispiel 1,
- unvermahlenem Flutamid, nicht intensiv gemischt mit Natriumdodecylsulfat (Beispiel 5),
- micronisiertem Flutamid, nicht intensiv gemischt mit Natriumdodecylsulfat (Beispiel 6), Mengenverhältnisse wie in Beispiel 5,
wurde bestimmt.

Apparatur zur Bestimmung der Wirkstoff-Freisetzung: Vankel-Apparatur mit Blattrührer
Zelle: 1000 ml
Medium: 2%-ige Natriumdodecylsulfat-Lösung
Temperatur: 37 °C
Rührgeschwindigkeit: 75 UpM

| | **Verarbeitung** | **Zeit /min.** | **Freigesetzter Wirkstoff in %** |
|---|---|---|---|
| **Beispiel 1** | Unvermahlenes Flutamid, intensiv gemischt | 30 | 100 |
| **Beispiel 2** | Micronisiertes Flutamid, nicht intensiv gemischt, gleiche Zusammensetzung wie in Beispiel 1 | 30 | 71 |
| **Beispiel 3** | Unvermahlenes Flutamid, intensiv gemischt | 60 | 93 |
| **Beispiel 4** | Unvermahlenes Flutamid, intensiv gemischt | 30 | 92 |
| **Beispiel 5** | Unvermahlenes Flutamid, nicht intensiv gemischt | 30 | 51 |
| **Beispiel 6** | Micronisiertes Flutamid, nicht intensiv gemischt, Mengenverhältnisse wie in Beispiel 5 | 30 | 87 |

Wie man der Tabelle entnehmen kann, ergibt sich für eine Formulierung mit unvermahlenem Flutamid, welches intensiv mit einer oberflächenaktiven Substanz gemischt wurde, die höchste Freisetzungsrate.

Beispiele 1 und 2 zeigen, daß die Verwendung von micronisiertem Flutamid ohne den erfindungsgemäßen, intensiven Mischprozess zu einer geringeren Freisetzungsrate führt als der Einsatz von unvermahlenem Flutamid, welches intensiv mit einer oberflächenaktiven Substanz gemischt wurde.

Beispiele 5 und 6 bestätigen die aus der Literatur bekannte Beobachtung, daß durch die Verwendung von micronisiertem Wirkstoff eine höhere Freisetzungsrate im Vergleich zu unvermahlenem Wirkstoff erzielt werden kann. Die Herstellung der Tabletten erfolgte nach bekannten Granulierverfahren ohne ein Zwangsmischen der Bestandteile.

## Patentansprüche

1. Verfahren zur Herstellung einer pharmazeutischen Formulierung mit kristallinem und/oder amorphem unvermahlenen Flutamid im Gemisch mit mindestens einer oberflächenaktiven Substanz, wobei die Größe von 50% der Flutamid-Teilchen (X50-Wert) in der pharmazeutischen Formulierung größer als 26 µm ist, bei dem man das Flutamid mit der mindestens einen oberflächenaktiven Substanz einem intensiven Mischprozess unter Verwendung eines Zwangsmischers unterwirft.

2. Verfahren nach Anspruch 1, wobei die Größe von 90% der Flutamid-Teilchen (X90-Wert) in der pharmazeutischen Formulierung größer als 60 µm ist.

3. Verfahren nach Anspruch 2, wobei die Größe von 90% der Flutamid-Teilchen (X90-Wert) in der pharmazeutischen Formulierung größer als 130 µm ist.

4. Verfahren nach einem der Ansprüche 1 bis 3, wobei Flutamid in Form des freien Säureamids und/oder eines pharmazeutisch unbedenklichen Solvats vorliegt.

5. Verfahren nach einem der Ansprüche 1 bis 4, wobei die mindestens eine oberflächenaktive Substanz aus der durch
- anionenaktive Verbindungen
- kationenaktive Verbindungen und
- nichtionogene Tenside
gebildeten Gruppe ausgewählt ist.

6. Verfahren nach einem der Ansprüche 1 bis 5 mit Natriumdodecylsulfat als oberflächenaktiver Substanz.

7. Verfahren nach einem der Ansprüche 1 bis 6 mit einem Gewichtsverhältnis Flutamid : oberflächenaktive Substanz(en) in der Formulierung von 5 : 1 bis 20 : 1.

8. Verfahren nach einem der Ansprüche 1 bis 7, bei dem man bei einer Temperatur von 0 bis 40 °C mischt.

9. Verfahren nach Anspruch 8, bei dem man bei Raumtemperatur mischt.

10. Verfahren nach einem der Ansprüche 1 bis 9, bei dem man ein oder mehrere Hilfsstoffe mit einem Zwangsmischer oder mit einem Freifallmischer zumischt.

11. Verfahren nach einem der Ansprüche 1 bis 10, bei dem man ein oder mehrere weitere pharmazeutische Wirkstoffe, bei denen es sich nicht um Flutamid handelt, mit einem Zwangsmischer oder mit einem Freifallmischer zumischt.

12. Verfahren nach mindestens einem der Ansprüche 1 bis 11, bei dem man die erhaltene Mischung zu Tabletten, Kapseln, Dragees, Brausetabletten, Zäpfchen oder Granulat weiterverarbeitet.

13. Formulierung hergestellt nach dem Verfahren von mindestens einem der vorhergehenden Ansprüche mit einem Gehalt an mindestens einem Hilfsstoff aus der durch anorganische Füllstoffe, organische Füllstoffe, Bindemittel, Gleitmittel, Schmiermittel, Fliessregulierungsmittel und/oder Sprengmittel gebildeten Gruppe.

14. Formulierung hergestellt nach dem verfahren von mindestens einem der vorhergehenden Ansprüche mit einem Gehalt an mindestens einem weiteren pharmazeutischen Wirkstoff neben Flutamid.

## Claims

1. Process for the preparation of a pharmaceutical formulation comprising crystalline and/or amorphous unmilled flutamide mixed with at least one surface-active substance, wherein the size of 50 % of the flutamide particles (X50 value) in the pharmaceutical formulation is greater than 26 µm, in which the flutamide is subjected to an intensive mixing process with the at least one surface-active substance by using a forced-action mixer.

2. Process according to claim 1, wherein the size of 90 % of the flutamide particles (X90 value) in the pharmaceutical formulation is greater than 60 µm.

3. Process according to claim 2, wherein the size of 90 % of the flutamide particles (X90 value) in the pharmaceutical formulation is greater than 130 µm.

4. Process according to any one of claims 1 to 3 wherein the flutamide is in the form of the free acid amide and/or of a pharmaceutically acceptable solvate.

5. Process according to any one of claims 1 to 4 wherein the at least one surface-active substance is selected from the group formed by
- anionic compounds,
- cationic compounds and
- non-ionic surfactants.

6. Process according to any one of claims 1 to 5 wherein the surface-active substance is sodium dodecylsulphate.

7. Process according to any one of claims 1 to 6 wherein the ratio by weight of flutamide : surface-active substance(s) in the formulation is from 5 : 1 to 20 : 1.

8. Process according to any one of claims 1 to 7 wherein mixing is carried out at a temperature of from 0 to 40°C.

9. Process according to claim 8 wherein mixing is carried out at room temperature.

10. Process according to any one of claims 1 to 9 wherein one or more excipients are admixed using a forced-action mixer or using a free-fall mixer.

11. Process according to any one of claims 1 to 10 wherein one or more further pharmaceutical active ingredients, which are not flutamide, are admixed using a forced-action mixer or a free-fall mixer.

12. Process according to at least one of claims 1 to 11 wherein the mixture obtained is further processed to form tablets, capsules, dragées, effervescent tablets, suppositories or a granulate.

13. Formulation produced according to the process of at least one of the preceding claims with a content of at least one excipient from the group formed by inorganic fillers, organic fillers, binders, glidants, lubricants, flow regulators and/or disintegrants.

14. Formulation produced according to the process of at least one of the preceding claims with a content of at least one further pharmaceutical active ingredient besides flutamide.

## Revendications

1. Procédé de préparation d'une formulation pharmaceutique avec du flutamide cristallin et/ou amorphe non moulu en mélange avec au moins une substance tensioactive, la taille de 50 % des particules de flutamide (valeur X₅₀) dans la formulation pharmaceutique étant supérieure à 26 µm, dans lequel on soumet le flutamide avec la au moins une substance tensioactive à un procédé de mélange intensif en utilisant un malaxeur à mélange forcé.

2. Procédé selon la revendication 1, dans lequel la taille de 90 % des particules de flutamide (valeur X₉₀) dans la formulation pharmaceutique est supérieure à 60 µm.

3. Procédé selon la revendication 2, dans lequel la taille de 90 % des particules de flutamide (valeur X₉₀) dans la formulation pharmaceutique est supérieure à 130 µm.

4. Procédé selon l'une des revendications 1 à 3, dans lequel le flutamide se présente sous la forme d'acétamide libre et/ou d'un solvate pharmaceutiquement acceptable.

5. Procédé selon l'une des revendications 1 à 4, dans lequel la au moins une substance tensioactive est choisie dans le groupe constitué par
les composés anioniques
les composés cationiques et
les tensioactifs non ionogènes.

6. Procédé selon l'une des revendications 1 à 5 comprenant du dodécylsulfate de sodium comme substance tensioactive.

7. Procédé selon l'une des revendications 1 à 6 ayant un rapport en poids entre flutamide / substance(s) tensioactive(s) dans la formulation de 5 : 1 à 20 : 1.

8. Procédé selon l'une des revendications 1 à 7, dans lequel on mélange à une température de 0 à 40°C.

9. Procédé selon la revendication 8, dans lequel on mélange à température ambiante.

10. Procédé selon l'une des revendications 1 à 9, dans lequel on ajoute un ou plusieurs excipients avec un malaxeur à mélange forcé ou un malaxeur à chute libre.

11. Procédé selon l'une des revendications 1 à 10, dans lequel on ajoute une ou plusieurs autres substances actives pharmaceutiques, qui ne sont pas du flutamide, avec un malaxeur à mélange forcé ou avec un malaxeur à chute libre.

12. Procédé selon l'une des revendications 1 à 11, dans lequel on retransforme le mélange obtenu en comprimés, gélules, capsules, tablettes à mâcher, suppositoires ou granulés.

13. Formulation préparée d'après le procédé selon au moins l'une des revendications précédentes, ayant une teneur en au moins un excipient choisi dans le groupe constitué par les charges inorganiques, les charges organiques, les liants, les agents de glissement, les lubrifiants, les agents de régulation de l'écoulement et/ou les agents de désintégration.

14. Formulation préparée d'après le procédé selon au moins l'une des revendications précédentes, ayant une teneur en au moins une autre substance active pharmaceutique en dehors du flutamide.
